(19) Europäisches Patentamt

European Patent Office

Office européen des brevets

(11) Numéro de publication : **0 128 166**
**B1**

(12) **FASCICULE DE BREVET EUROPÉEN**

(45) Date de publication du fascicule du brevet :
**07.05.86**

(21) Numéro de dépôt : **83903802.3**

(22) Date de dépôt : **07.12.83**

(86) Numéro de dépôt international :
**PCT/FR 83/00244**

(87) Numéro de publication internationale :
**WO/8402349 (21.06.84 Gazette 84/15)**

(51) Int. Cl.⁴ : **C 12 M 1/00**

(54) **DIGESTEUR CONTINU POUR LA PRODUCTION DE BIOMETHANE A PARTIR DE SUBSTANCES ORGANIQUES.**

(30) Priorité : **08.12.82 FR 8220809**

(43) Date de publication de la demande :
**19.12.84 Bulletin 84/51**

(45) Mention de la délivrance du brevet :
**07.05.86 Bulletin 86/19**

(84) Etats contractants désignés :
**AT BE CH DE GB LI LU NL SE**

(56) Documents cités :
**DE-C- 884 176**
**FR-A- 2 419 322**
**US-A- 2 538 412**
**US-A- 4 046 551**

(73) Titulaire : **Guerin, Maurice**
**53 rue Victor Hugo**
**F-71000 Macon (FR)**

**Ribaud, Patricia Catherine**
**20 rue Emile Dubois**
**F-75014 Paris (FR)**

(72) Inventeur : **Guerin, Maurice**
**53 rue Victor Hugo**
**F-71000 Macon (FR)**
Inventeur : **Ribaud, Patricia Catherine**
**20 rue Emile Dubois**
**F-75014 Paris (FR)**

(74) Mandataire : **Casalonga, Alain et al**
**BUREAU D.A. CASALONGA OFFICE JOSSE & PETIT**
**Baaderstrasse 12-14**
**D-8000 München 5 (DE)**

## Description

La présente invention concerne les appareils destinés à produire en continu du biométhane à partir de substances organiques contenues dans des déchets animaux ou végétaux provenant essentiellement de l'agriculture ou des industries dérivées de celle-ci.

Elle concerne également les systèmes d'épuration des effluents fortement chargés de matières organiques puisque la fermentation méthanogène s'accompagne d'une forte réduction de la D B O$_5$.

Dans l'état actuel de la technique, on maîtrise mal l'hydraulique interne des digesteurs continus. L'efficacité des systèmes de brassage utilisés est douteuse ; ils présentent en outre généralement des inconvénients d'ordre mécanique ou biologique. On ignore comment se répartissent les produits entrants dans le volume des produits en cours de digestion.

La présente invention vise à remédier aux inconvénients de la technique antérieure et propose un appareil pour la production de biométhane à partir de substances organiques tel que toute partie supérieure du digesteur et son pilier central d'appui dont elle est solidaire mécaniquement sont mobiles en rotation autour de l'axe central vertical de la cuve, que la partie supérieure du digesteur prend appui par l'intermédiaire de galets à la face supérieure des parois latérales et sur le pilier central qui lui-même prend appui sur le plancher de la cuve par l'intermédiaire d'une crapaudine, que la partie centrale de la cuve est occupée par un compartiment cylindrique appelé compartiment primaire et dont l'axe est confondu avec l'axe de la cuve du digesteur, que la cuve est traversée par une cloison fixe disposée selon un rayon de la cuve, qu'une cloison mobile liée rigidement à la partie supérieure du digesteur et au pilier central effectue des aller et retour circulaires de façon à balayer tout le volume de la cuve en dehors du compartiment primaire, que à la partie supérieure de la cloison mobile sont fixées une pompe de recirculation et une pompe d'introduction. Pendant le déplacement de la cloison mobile la pompe de recirculation faisant passer de part et d'autre de la cloison tout le volume balayé pendant le déplacement et simultanément la pompe d'introduction introduisant les produits en provenance de la base du compartiment primaire dans la veine des produits en cours de déplacement sous l'action de la pompe de recirculation et que le biogaz est recueilli par une bâche souple solidaire de la partie supérieure mobile du digesteur.

L'appareil selon la présente invention comprend, de préférence, une partie supérieure mobile dont les points d'appuis sont tels qu'ils permettent la rotation dans les deux sens de la partie supérieure du digesteur et des éléments qui lui sont liés directement ou indirectement autour de l'axe central vertical de la cuve, que les poutrelles qui supportent directement ou indirectement toutes les parties mobiles prennent appui à une de leur extrémité sur la partie supérieure du pilier central et qu'elles sont reliées rigidement à lui, qu'à leur autre extrémité chacune d'elle est reliée à un galet à axe horizontal qui prend appui sur la face supérieure des parois latérales et à un galet à axe vertical qui les maintient dans un plan vertical et qu'une crapaudine fixée dans la dalle sert de guide pour les mouvements de rotation et de butée pour les efforts verticaux du pilier support.

L'appareil selon la présente invention comporte de préférence une cloison fixe. Cette cloison fixe obture complètement la section déterminée par : en haut, le fond des canaux d'entrée et de sortie des produits ; en bas, le plancher chauffant vers l'extérieur la paroi latérale et vers l'intérieur la paroi du compartiment primaire.

L'appareil selon la présente invention comporte de préférence une paroi mobile telle que cette paroi est suspendue à la poutrelle qui est située au-dessus d'elle, qu'un bras de liaison relie le bas de la paroi mobile à la base du pilier par une liaison rigide, et que la paroi mobile balaye tout le volume situé de part et d'autre de la paroi fixe en se déplaçant tantôt dans un sens tantôt dans l'autre.

L'appareil selon la présente invention comprend, de préférence, un ensemble de pompes et de canalisations tel que la pompe d'introduction est reliée à une canalisation d'aspiration dont l'origine se situe à la base du compartiment primaire, que la pompe d'introduction dirige les produits en provenance de la base du compartiment primaire soit à la partie supérieure du compartiment primaire, soit dans le courant des produits mis en mouvement par la pompe de recirculation, que lorsque la paroi mobile se déplace dans le sens des aiguilles d'une montre les produits sont prélevés par la pompe de recirculation devant la paroi et au pied de celle-ci et sont rejetés derrière la paroi et en haut, de façon symétrique lorsque la paroi se déplace en sens inverse des aiguilles d'une montre les produits sont prélevés par cette même pompe de recirculation et rejetés, et que la canalisation de refoulement et son extrémité sont disposées de telle façon qu'en fin de parcours de la paroi mobile elles viennent au-dessus du début du canal de sortie après avoir franchi l'échancrure.

La présente invention sera mieux comprise à l'étude d'un appareil pour la production de biométhane à partir de substances organiques décrit à titre d'exemple non limitatif et illustré par les figures sur lesquelles :

la figure 1 est une coupe verticale passant par l'axe central de la cuve et par deux galets de suspension de la partie supérieure situés de part et d'autre de l'axe central ;

la figure 2 est une coupe verticale partielle passant par le début du canal de sortie ;

la figure 3 est une vue en perspective cavalière montrant la disposition de la fin du canal d'entrée et le début du canal de sortie par rapport à la cloison fixe ;

la figure 4 est une coupe verticale partielle passant par l'axe central de la cuve et par la paroi mobile ;

la figure 5 est une coupe verticale transversale à travers la paroi mobile ; et

la figure 6 est une coupe horizontale faite à travers le digesteur, le plan de coupe passant en dessous du bord latéral de la bâche de récupération du biogaz.

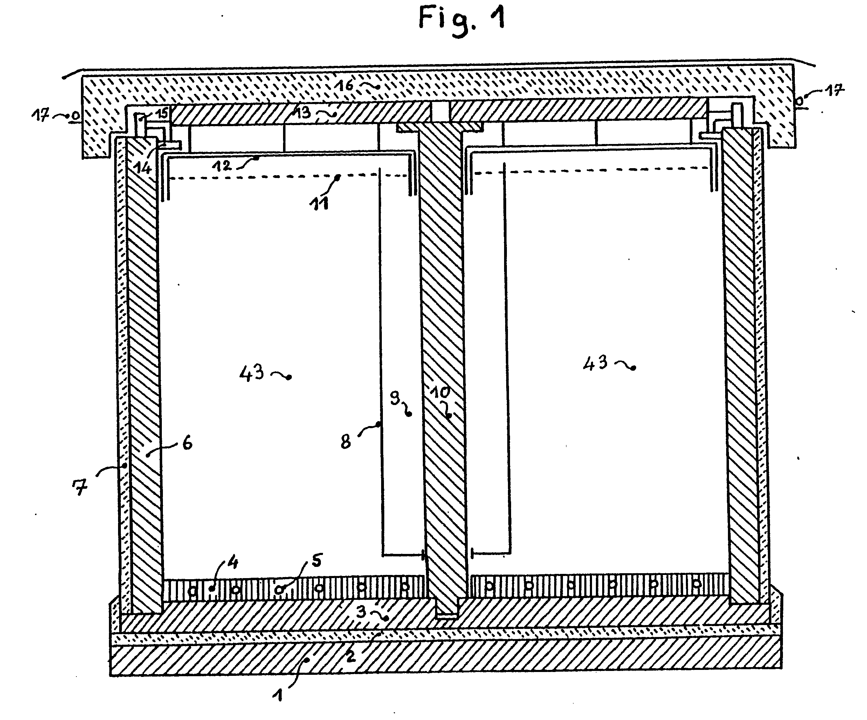

Sur la figure 1, on voit que la base du digesteur est constituée de bas en haut et successivement d'un socle en béton 1, d'une couche continue d'isolant incompressible 2, d'une dalle en béton 3, et d'un plancher chauffant 4 parcouru par des tubes de chauffage 5. La paroi latérale cylindrique 6 est revêtue à l'extérieur d'une couche continue d'isolant 7.

Toute la partie supérieure du digesteur et son pilier central d'appui 10 dont elle est solidaire mécaniquement sont mobiles en rotation autour de l'axe central de la cuve. Pour permettre ceci, au centre de la dalle 3 est fixée une crapaudine qui reçoit l'extrémité inférieure du pilier support 10 et lui permet de faire des mouvements circulaires alternatifs en servant de guide pour les mouvements de rotation et de butée pour les efforts verticaux.

A la partie supérieure, la bâche souple 12 de récupération du biogaz est solidaire de la partie mobile du digesteur. Les bords latéraux de la bâche 12 plongent dans les produits dont le niveau est représenté par le pointillé 11.

Les poutrelles support 15 soutiennent la bâche 12 de récupération du biogaz, au-dessus d'elles on a une couche continue d'isolant 16 surmontée d'une couverture.

Les poutrelles 13 qui supportent directement ou indirectement toutes les parties mobiles et en particulier toute la partie supérieure du digesteur prennent appui à une de leur extrémité sur la partie supérieure du pilier central 10 auquel elles sont liées de façon rigide, à leur autre extrémité, chacune d'elles est reliée à un galet 15 à axe horizontal qui la soutient horizontalement et à un galet 14 à axe vertical qui la guide verticalement. L'ensemble pilier central 10 galet 15 et galet 14 permet la rotation dans les deux sens de la partie supérieure du digesteur et des éléments qui lui sont liés.

Le câble 17 ceinture la face latérale verticale externe au niveau de l'isolant 16. Ce câble est amarré en un point de cette face latérale et il est relié à un dispositif d'entraînement réversible.

La partie centrale de la cuve est occupée par le compartiment primaire 9 dont la paroi latérale 8 est cylindrique et a le même axe vertical que le pilier support 10 et que l'axe de la cuve du digesteur.

Le reste du volume de la cuve est occupé par le compartiment secondaire 43.

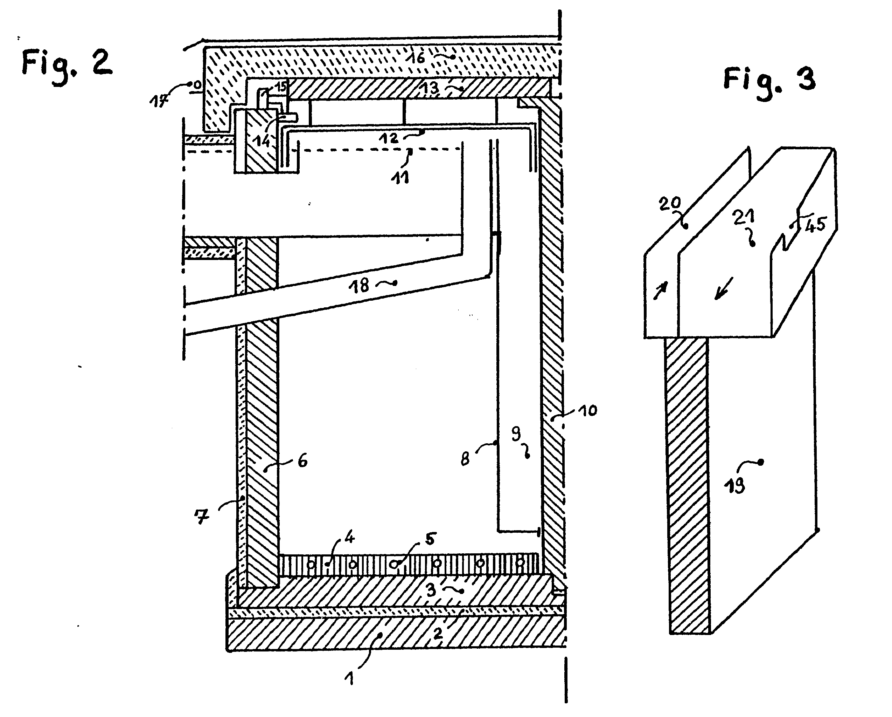

Sur la figure 2, on voit que la canalisation 18 d'évacuation du biogaz débute par un tube vertical qui traverse de haut en bas près de la paroi 8 du compartiment primaire le début du canal de sortie 21 puis se continue par un tube incliné qui après avoir traversé la paroi latérale 6 débouche à l'extérieur.

Sur la figure 3, on a représenté en 19 la cloison fixe. Cette cloison fixe 19 disposée selon un rayon de la cuve obture complètement la section déterminée par : en haut, le fond de la fin 20 du canal d'entrée, le fond du début 21 du canal de sortie ; en bas, le plancher chauffant 4 ; vers l'extérieur la paroi latérale 6 et vers l'intérieur la paroi 8 du compartiment primaire 9 (les éléments 4, 6, 8, 9 sont représentés sur la figure 2).

La cloison fixe 19 soutient la fin du canal d'entrée et le début 21 du canal de sortie ; celui-ci est alimenté par une échancrure 45 pratiquée dans la paroi latérale du canal en dessous du niveau 11 des produits dans le digesteur (le niveau 11 est représenté sur la figure 2).

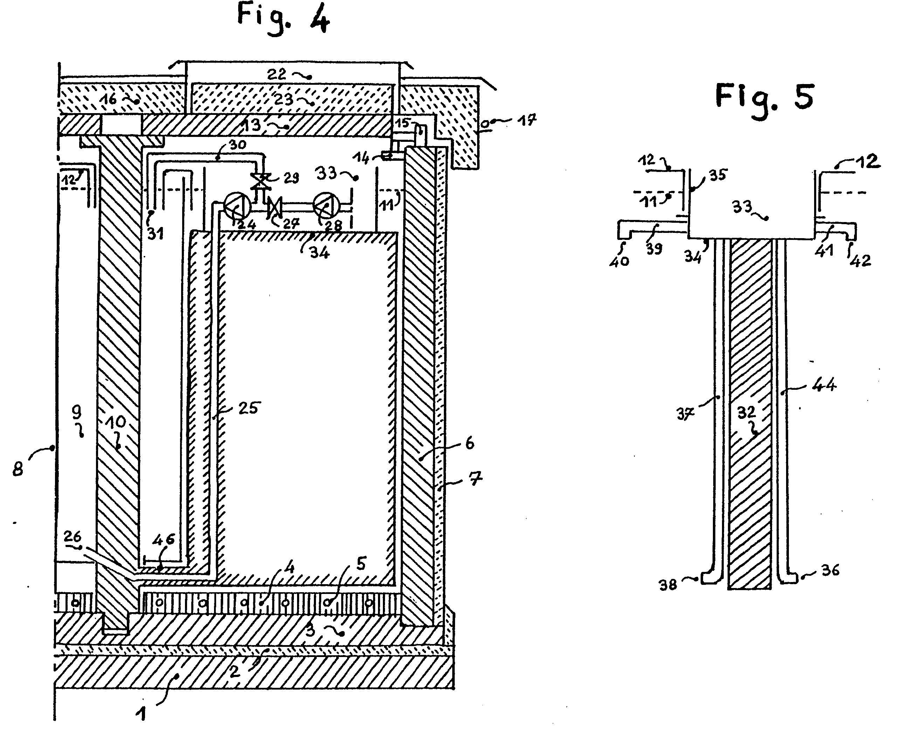

Sur la figure 4, on voit que le bras de liaison 46 relie de façon rigide le bas de la paroi mobile à la base du pilier mobile en rotation 10. Pour permettre cette liaison et le mouvement de rotation alternatif du bras 46 le fond du compartiment primaire est à une distance suffisante du plancher chauffant 4.

A la partie supérieure de la paroi mobile on a un bac 33 ouvert à sa partie supérieure et partiellement immergé dans la partie supérieure des produits. Le fond 34 de ce bac est horizontal et il est à un niveau tel que les pompes d'introduction 24 et de recirculation 28 qui sont à l'intérieur du bac soient en charge.

La pompe d'introduction 24 est reliée à la canalisation d'aspiration 25, l'origine 26 de cette canalisation se situe à la base du compartiment primaire 9.

La pompe d'introduction 24 dirige les produits en provenance de la base du compartiment primaire : soit à la partie supérieure du compartiment primaire par l'intermédiaire de la vanne 29 et de la canalisation 30 qui débouche en 31, soit par l'intermédiaire de la vanne 27 dans le courant des produits mis en mouvement par la pompe de recirculation 28.

La paroi mobile qui est en dessous du bac 33 est suspendue à la poutrelle 13 qui est située au-dessus d'elle, elle est reliée rigidement à la partie supérieure mobile du digesteur et son axe de rotation est dont l'axe du pilier 10.

Au-dessus du bac 33 une ouverture rectangulaire 22 traverse la couverture et toute la couche d'isolant 16, cette ouverture 22 est obturée par un tampon isolant amovible 23 surmonté d'une ouverture amovible.

Au-dessus du bac 33 une ouverture rectangulaire 22 traverse la couverture et toute la couche d'isolant 16, cette ouverture 22 est obturée par un tampon amovible 23 surmonté d'une couverture amovible.

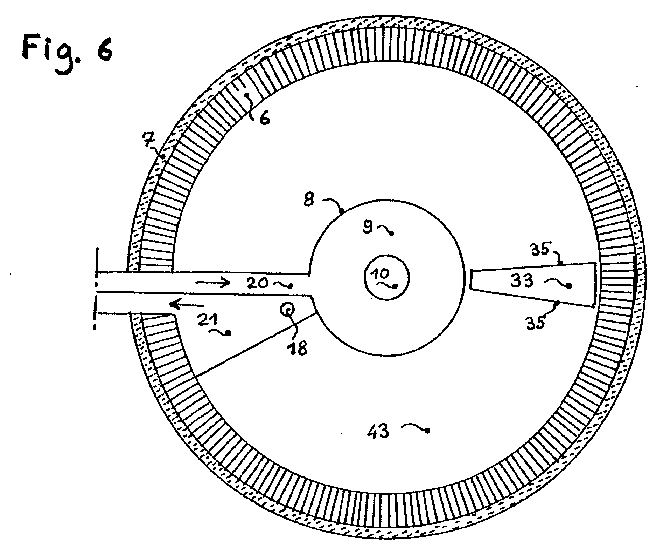

Sur la figure 5, on voit que la paroi mobile 32 effectue des aller et retour circulaires de façon à balayer tout le volume de la cuve en dehors du compartiment primaire. Ce volume 43 (voir figure 6) est situé de part et d'autre de la cloison fixe 19 (voir figure 3).

Le bac 33 est fixé par sa paroi du fond 34 à la partie supérieure de la paroi mobile. La bâche souple 12 de récupération du biogaz vient s'accoler contre les parois latérales 35 du bac.

La canalisation d'aspiration 37 est située d'un côté de la paroi mobile 32 et elle a son origine en 38 au pied de la paroi mobile. L'autre canalisation d'aspiration 44 est de l'autre côté de la paroi et son origine 36 est au pied de la paroi.

Les canalisations de refoulement 39 et 41 sont situées de part et d'autre de la paroi mobile à la partie supérieure.

Elles débouchent en 40 et 42 un peu en dessous du niveau 11 des produits.

Lorsque la paroi mobile 32 se déplace dans le sens des aiguilles d'une montre les produits sont prélevés par la pompe de recirculation 28 devant la paroi et au pied de celle-ci en 38 et sont rejetés derrière la paroi et en haut en 42.

De façon symétrique lorsque la paroi se déplace en sens inverse des aiguilles d'une montre les produits sont prélevés par cette même pompe de recirculation 28 en 36 et rejetés en 40.

La canalisation de refoulement 39 et son extrémité 40 sont disposées de telle façon qu'en fin de parcours de la paroi mobile 32 elles viennent au-dessus du début 21 (figure 3) du canal de sortie après avoir franchi l'échancrure 45 (figure 3).

Sur la figure 6, on voit qu'à l'intérieur du digesteur le canal d'entrée 20 est contigu sur tout son parcours au canal de sortie 21.

Le canal d'entrée 20 pénètre dans le digesteur par une ouverture pratiquée dans la paroi latérale 6 ; cette ouverture a la même section que le canal, la partie terminale du canal d'entrée se jette dans le compartiment primaire par une échancrure de même section que le canal d'entrée.

Le canal de sortie débute à l'intérieur du digesteur, il sort du digesteur par une ouverture pratiquée dans la paroi latérale 6, ouverture de même section que celle permettant le passage du canal d'entrée.

On a représenté en position quelconque le bac 33, ses parois latérales 35 et la paroi mobile qui est située en dessous.

L'appareil ci-dessus décrit fonctionne de la manière suivante.

Les produits frais sont introduits par le canal d'entrée 20. Ce canal est alimenté en refus par une pompe et une lame déversante située au début du canal y maintient le niveau des produits constants.

Le canal d'entrée 20 débouche à la partie supérieure du compartiment primaire 9.

Les produits entrants séjournent environ 36 heures dans le compartiment primaire ; ils y subissent des réactions de dépolymérisation et d'acidification.

Les produits sont ensuite prélevés à la base du compartiment primaire par la pompe d'introduction 24 qui les envoie alternativement : à la partie supérieure du compartiment primaire 9 pour ensemencer les produits entrants, dans la veine des produits en cours de recirculation grâce à la pompe de recirculation 28.

La paroi mobile 32 se déplace alternativement dans un sens puis dans l'autre de part et d'autre de la paroi fixe 19. Au cours de chaque déplacement, elle balaye l'ensemble du volume du compartiment secondaire 43.

Pendant ces mouvements et dans chaque sens, la pompe de recirculation aspire les produits devant la paroi mobile en bas de celle-ci et les refoule derrière cette même paroi mobile et en haut de celle-ci un peu en dessous du niveau général des produits 11.

Le débit de la pompe de recirculation 28 est tel que pendant un temps donné il est égal au volume balayé par la paroi mobile 32 pendant ce même temps.

Lorsque la paroi mobile 32 arrive près de la paroi fixe 19 du côté du début 21 du canal de sortie l'extrémité 40 de la canalisation 39 vient au-dessus du début du canal de sortie 21 grâce à l'échancrure 45.

A ce moment la pompe de recirculation 28 aspire en 38 grâce à la canalisation 37 et refoule du même côté dans la canalisation 39 ce qui permet d'envoyer dans le canal de sortie les boues qui se sont accumulées devant la paroi mobile 32.

Dans le compartiment secondaire 43 se réalisent les réactions de digestion anaérobie productrices de méthane.

Lorsque la paroi mobile s'approche du début 21 du canal de sortie, on arrête la pompe d'introduction 24 de façon qu'il n'y ait pas de produits non digérés dans les produits qui quittent le digesteur.

Les produits quittent le digesteur en parcourant le canal de sortie 21.

Ce canal se termine par une lame déversante qui maintient donc automatiquement les produits au même niveau constant dans le canal de sortie et dans le compartiment secondaire.

Les produits sont réchauffés et maintenus à la température de digestion par le plancher chauffant 4.

Le biogaz est recueilli à la surface des produits par la bâche souple 12 dont les bords latéraux plongent dans les produits assurant ainsi l'étanchéité par un joint hydraulique. Le biogaz quitte le digesteur par une canalisation 18.

La traction par un système moteur sur le câble 17 tantôt dans un sens tantôt dans l'autre réalise la rotation dans un sens tantôt dans l'autre de toute la partie supérieure, du pilier central d'appui 10 et de la paroi mobile 32.

Les avantages apportés par l'invention sont la disparition totale des problèmes posés par la croûte et par la sédimentation des boues au fond du digesteur, ainsi que l'apport régulier et homogène de nutriments dans toute la masse des produits en cours de digestion, ce qui est un facteur très important pour le rendement et ce qui permet une bonne utilisation du pouvoir tampon des produits en cours de fermentation.

## Revendications

1. Appareil pour la production de biométhane à partir de substances organiques caractérisé :

en ce que toute la partie supérieure du digesteur et son pilier central d'appui (10) dont elle est solidaire mécaniquement sont mobiles en rotation autour de l'axe central vertical de la cuve,

en ce que la partie supérieure du digesteur prend appui par l'intermédiaire de galets (15) à la face supérieure des parois latérales et sur le pilier central (10) qui lui-même prend appui sur le plancher de la cuve par l'intermédiaire d'une crapaudine,

en ce que la partie centrale de la cuve est occupée par un compartiment cylindrique (9) appelé compartiment primaire et dont l'axe est confondu avec l'axe de la cuve du digesteur,

en ce que la cuve est traversée par une cloison fixe (19) disposée selon un rayon de la cuve,

en ce qu'une cloison mobile (32) liée rigidement à la partie supérieure du digesteur et au pilier central (10) effectue des aller et retour circulaires de façon à balayer tout le volume de la cuve en dehors du compartiment primaire,

en ce qu'à la partie supérieure de la cloison mobile sont fixées une pompe de recirculation (28) et une pompe d'introduction (24) pendant le déplacement de la cloison mobile la pompe de recirculation (28) faisant passer de part et d'autre de la cloison tout le volume balayé pendant le déplacement et simultanément la pompe d'introduction (24) introduit les produits en provenance de la base du compartiment primaire dans la veine des produits en cours de déplacement sous l'action de la pompe de recirculation (28),

en ce que le biogaz est recueilli par une bâche souple (12) solidaire de la partie supérieure mobile du digesteur.

2. Appareil selon la revendication 1 comprenant une partie supérieure mobile dont les points d'appui sont caractérisés :

en ce qu'ils permettent la rotation dans les deux sens de la partie supérieure du digesteur et des éléments qui lui sont liés directement ou indirectement autour de l'axe central vertical de la cuve,

en ce que les poutrelles (13) qui supportent directement ou indirectement toutes les parties mobiles prennent appui à une de leur extrémité sur la partie supérieure du pilier central (10) et qu'elles sont reliées rigidement à lui, qu'à leur autre extrémité chacune d'elles est reliée à un galet (15) à axe horizontal qui prend appui sur la face supérieure des parois latérales et à un galet (14) à axe vertical qui les maintient dans un plan vertical,

en ce qu'une crapaudine fixée dans la dalle (3) sert de guide pour les mouvements de rotation et de butée pour les efforts verticaux du pilier support (10).

3. Appareil selon la revendication 1 comportant une cloison fixe (19) caractérisée en ce que cette cloison fixe (19) obture complètement la section déterminée par : en haut, le fond des canaux d'entrée (20) et de sortie (21) des produits ; en bas, le plancher chauffant (4) vers l'extérieur la paroi latérale (6) et vers l'intérieur la paroi (8) du compartiment primaire.

4. Appareil selon la revendication 1 comprenant une paroi mobile (32) caractérisée :

en ce que cette paroi mobile (32) est suspendue à la poutrelle (13) qui est située au-dessus d'elle,

en ce qu'un bras de liaison (46) relie le bas de la paroi mobile à la base du pilier (10) par une liaison rigide,

en ce que la paroi mobile (32) balaye tout le volume situé de part et d'autre de la paroi fixe (19) en se déplaçant tantôt dans un sens tantôt dans l'autre.

5. Appareil selon la revendication 1 comprenant un ensemble de pompes et de canalisations caractérisées :

en ce que la pompe d'introduction (24) est reliée à une canalisation (25) d'aspiration dont l'origine (26) se situe à la base du compartiment primaire,

en ce que la pompe d'introduction (24) dirige les produits en provenance de la base du compartiment primaire : soit à la partie supérieure du compartiment primaire, soit dans le courant des produits mis en mouvement par la pompe de recirculation (28),

en ce que lorsque la paroi mobile (32) se déplace dans le sens des aiguilles d'une montre les produits sont prélevés par la pompe de recirculation (28) devant la paroi et au pied de celle-ci en (38) et sont rejetés derrière la paroi et en haut en (42), de façon symétrique lorsque la paroi se déplaçant en sens inverse des aiguilles d'une montre les produits étant prélevés par cette même pompe de recirculation (28) en (36) et rejetés en (40),

en ce que la canalisation de refoulement (39) et son extrémité sont disposées de telle façon qu'en fin de parcours de la paroi mobile (32) elles viennent au-dessus du début (21) du canal de sortie après avoir franchi l'échancrure (45).

## Claims

1. Apparatus for producing biomethane from organic substances, characterized :

in that the entire upper part of the digester and its central support pillar (10), to which it is mechanically fixed, are rotationally movable about the vertical centre axis of the vessel,

in that the upper part of the digester is supported by means of rollers (15) on the top face of the side walls and on the central pillar (10), which in turn is supported on the floor of the vessel by means of a footstep bearing,

in that the central part of the vessel is occupied by a cylindrical compartment (9) known as the primary compartment and whose axis is identical with the axis of the digester vessel,

in that a fixed partition (19) disposed on a radius of the vessel extends through the latter,

in that a movable partition (32) rigidly connected to the upper part of the digester and to the central pillar (10) makes circular reciprocating movements in such a manner as to sweep over the entire volume of the vessel apart from the primary compartment,

in that a recirculation pump (28) and an introduction pump (24) are fixed to the upper part of the movable partition, the recirculation pump (28) causing, during the displacement of the movable partition, the entire volume swept over during the displacement to pass on each side of the partition, while at the same time the introduction pump (24) introduces the products coming from the base of the primary compartment into the stream of products undergoing displacement under the action of the recirculation pump (28),

in that the biogas is collected by a flexible container (12) fastened to the movable upper part of the digester.

2. Apparatus according to Claim 1, comprising a movable upper part whose support points are characterized :

in that they permit the rotation in both directions of the upper part of the digester and of the elements directly or indirectly connected to it, about the vertical centre axis of the vessel,

in that the beams (13) which directly or indirectly support all the movable parts are supported at one end on the top part of the central pillar (10) and that they are rigidly connected to the latter, while at the other end each of them is connected to a roller (15) having a horizontal axis and supported on the top face of the side walls and to a roller (14) having a vertical axis and supporting them in a vertical plane,

in that a footstep bearing fixed in the floor slab (3) serves as guide for the rotary movement and as a thrust bearing for the vertical forces of the support pillar (10).

3. Apparatus according to Claim 1, comprising a fixed partition (19), characterized in that this fixed partition (19) completely closes the section determined by : at the top, the bottom of the inlet duct (20) and outlet duct (21) for the products ; at the bottom, the heating floor (4) ; towards the outside, the side wall (6), and towards the interior the wall (8) of the primary compartment.

4. Apparatus according to Claim 1, comprising a movable wall (32), characterized :

in that this movable wall (32) is suspended on the beam (13) which is situated above it,

in that a connecting arm (46) connects the bottom of the movable wall to the base of the pillar (10) by a rigid connection,

in that the movable wall (32) sweeps over the entire volume situated on each side of the fixed wall (19) in its movement first in one direction and then in the other.

5. Apparatus according to Claim 1, comprising an assembly of pumps and pipes, characterized :

in that the introduction pump (24) is connected to a suction pipe (25) whose starting point (26) is situated at the base of the primary compartment,

in that the introduction pump (24) directs the products coming from the base of the primary compartment either to the upper part of the primary compartment or into the stream of products caused to move by the recirculation pump (28),

in that when the movable wall (32) moves in the clockwise direction the products are taken up by the recirculation pump (28) in front of the wall and at the foot of the latter at (38) and are discharged behind the wall and at the top at (42), in a symmetrical manner when the wall moves in the counterclockwise direction the products are taken up by the same recirculation pump (28) at (36) and discharged at (40),

in that the delivery pipe (39) and its end are disposed in such a manner that at the end of the travel of the movable wall (32) they lie above the beginning (21) of the outlet duct after having cleared the cutout (45).


**Patentansprüche**

1. Vorrichtung zum Erzeugen von Biomethan aus organischen Stoffen, dadurch gekennzeichnet,

daß der ganze obere Teil des Digestors und sein Mittelstützpfeiler (10), mit dem er mechanisch fest verbunden ist, um die vertikale Mittelachse des Behälters drehbeweglich sind,

daß sich der obere Teil des Digestors über Rollen (15) an der oberen Fläche der Seitenwände und am Mittelpfeiler (10) abstützt, der sich seinerseits über ein Zapfenlager am Boden des Behälters abstützt,

daß der mittlere Teil des Behälters durch einen zylindrischen Raum (9), genannt erster Raum, eingenommen wird, und dessen Achse mit der Achse des Digestors identisch ist,

daß der Behälter von einer festen Wand durchquert wird, die längs eines Radius des Behälters ausgerichtet ist,

daß eine bewegliche Wand (32), die starr mit dem oberen Teil des Digestors und dem Mittelpfeiler (10) verbunden ist, kreisförmige Hin- und Herbewegungen ausführt, um das ganze Volumen des Behälters außerhalb des ersten Raumes zu bestreichen,

daß am oberen Teil der beweglichen Wand eine Umwälzpume (28) und eine Zuführpumpe (24) befestigt sind, wobei die Umwälzpumpe (28) während der Bewegung der beweglichen Wand beiderseits der Wand das während der Bewegung bestrichene Volumen passieren läßt und die Zuführpumpe (24) gleichzeitig die vom unteren Teil des ersten Raumes kommenden Stoffe in den Strom der sich unter der Wirkung der Umwälzpumpe (28) verlagernden Stoffe einführt und

und daß das Biogas über einen mit dem oberen, beweglichen Teil des Digestors verbundenen flexiblen Topf (12) gesammelt wird.

2. Vorrichtung nach Anspruch 1 mit einem oberen, beweglichen Teil, dessen Abstützpunkte dadurch gekennzeichnet sind,

daß sie die Drehung oberen Teils des Digestors und der mit diesem direkt oder indirekt verbundenen Bauteile in beiden Richtungen um die vertikale Mittelachse des Behälters erlauben,

daß sich die Träger (13), die direkt oder indirekt alle beweglichen Teile tragen, mit ihrem einen Ende am oberen Teil des Mittelpfeilers (10) abstützen und mit diesem starr verbunden sind, daß das andere Ende jedes von ihnen mit einer Rolle (15) mit horizontaler Achse, die sich auf der oberen Fläche der Seitenwände abstützt, und mit einer Rolle (14) mit vertikaler Achse, die sie in einer Vertikalebene hält, verbunden ist, und

daß ein in der Platte (3) befestigtes Zapfenlager zur Führung der Drehbewegungen und als Widerlager für Vertikalkräfte des Stützpfeilers (10) dient.

3. Vorrichtung nach Anspruch 1 mit einer festen Wand (19), dadurch gekennzeichnet, daß die feste Wand (19) vollständig den durch : oben den Boden des Eintrittskanals (20) und des Auslaßkanals (21) für die Stoffe ; unten den Heizboden (4), nach außen die Seitenwand (6) und nach innen die Wand (8) des ersten Behälters definierten Querschnitt verschließt.

4. Vorrichtung nach Anspruch 1 mit einer beweglichen Wand (32), dadurch gekennzeichnet,

daß die bewegliche Wand (32) an dem über ihr angeordneten Träger (13) aufgehängt ist,

daß ein Verbindungsarm (46) das untere Ende der beweglichen Wand mit dem unteren Ende des Pfeilers (10) durch eine starre Verbindung verbindet,

daß die bewegliche Wand (32) das gesamte, auf beiden Seiten der festen Wand (19) angeordnete Volumen bestreicht, wenn sie sich abwechselnd in der einen und in der anderen Richtung bewegt.

5. Vorrichtung nach Anspruch 1 mit einer Pumpenanordnung und Leitungen, dadurch gekennzeichnet,

daß die Zuführpumpe (24) an eine Ansaugleitung (25) angeschlossen ist, deren Anfang (26) im unteren Teil des ersten Raumes angeordnet ist,

daß die Zuführpumpe (24), die vom unteren Teil des Behälters kommenden Stoffe entweder zum oberen Teil des ersten Raumes oder in den Strom der durch die Umwälzpumpe (28) bewegten Stoffe lenkt,

daß die Stoffe, während sich die bewegliche Wand (32) im Uhrzeigersinn bewegt, durch die Umwälzpumpe (28) vor der Wand und am Boden dieser bei (38) abgezogen und hinter der Wand und oben bei (42) abgegeben werden, und in symmetrischer Weise, wenn sich die Wand im Gegenuhrzeigersinn bewegt, werden die Stoffe durch eben diese Umwälzpumpe (28) bei (36) angesaugt und bei (40) abgegeben, und daß die Verdrängungsleitung (39) und ihr Ende so angeordnet ist, daß sie am Ende der Bewegung der beweglichen Wand (32) über den Anfang (21) des Auslaßkanals anlangen, nachdem sie die Ausnehmung (45) übergriffen haben.

Fig. 1

Fig. 2

Fig. 3

0 128 166

# Fig. 4

# Fig. 5

0 128 166

Fig. 6

0 128 166